(19) Europäisches Patentamt
European Patent Office
Office européen des brevets

(11) Publication number: 0 301 257 A2

(12) # EUROPEAN PATENT APPLICATION

(21) Application number: 88110326.1

(51) Int. Cl.[4]: C07D 501/22 , A61K 31/545

(22) Date of filing: 29.06.88

Claims for the following Contracting States: ES + GR.

(30) Priority: 01.07.87 KR 876785

(43) Date of publication of application: 01.02.89 Bulletin 89/05

(84) Designated Contracting States: AT BE CH DE ES FR GB GR IT LI LU SE

(71) Applicant: KOREA RESEARCH INSTITUTE OF CHEMICAL TECHNOLOGY
100 Chang Dong Chung Ku, Daejeon
Chungnam 300-31(KR)

(72) Inventor: Kim, Wan-Joo
33-11, Sambu Apt Taepyeong-Dong
Jung-Ku Daejeon(KR)
Inventor: Kim, Hong-Bum
529-3, Mok-2-Dong
Yangcheonku Seoul(KR)
Inventor: Ko, Kwang-Youn
8-301, Kongdong Apt 431 Doryong-Dong
Seoku Daejeon(KR)
Inventor: Shim, Young-Key
8-503, Kongdong Apt, 431 Doryong-Dong
Seoku Daejeon(KR)
Inventor: Oh, Jong-Hoon
150, Singseoung-Dong
Seoku Daejeon(KR)

(74) Representative: Aulmich, Gerhard et al
Hoechst AG Zentrale Patentabteilung
Postfach 80 03 20
D-6230 Frankfurt am Main 80(DE)

(54) **Cephem Derivatives and a process for their manufacture.**

(57) Cephem derivatives of the formula

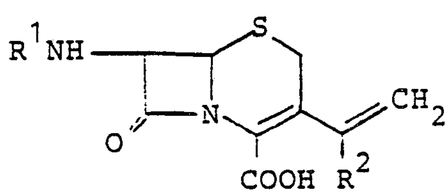

and physiologically acceptable salts thereof, a process for their manufacture, pharmaceutical preparations containing them and the intermediates of the formula

EP 0 301 257 A2

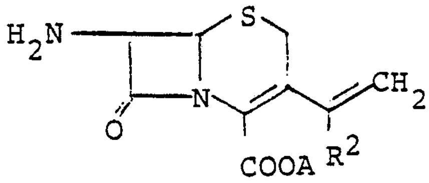

## Cephem Derivatives and a Process for their Manufacture

The present invention relates to new 10-substituted-3-vinyl cephem derivatives of the following formula

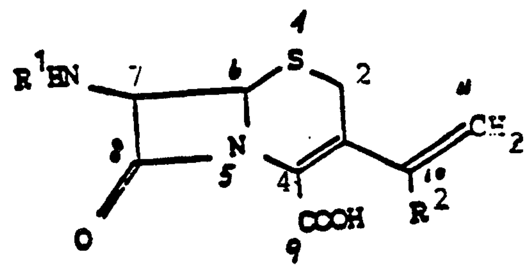

which are useful especially as oral antibiotics, to a process for their manufacture and to preparations containing these compounds.

Recently, there has been a rapid growth in the field of antibiotics, especially, regarding 3-vinyl cephalosporines. For example, U.S. 4,409,214 discloses a new cephalosporine derivative, cefixime, covered by the following formula

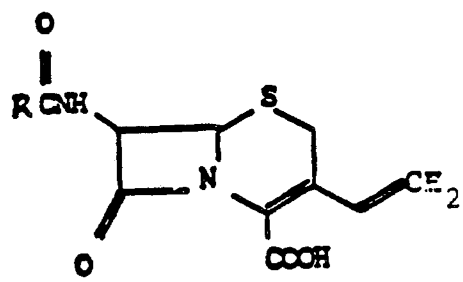

and U.S. 4,520,022 discloses another new cephalosporine, BMY-28100, covered by the following formula

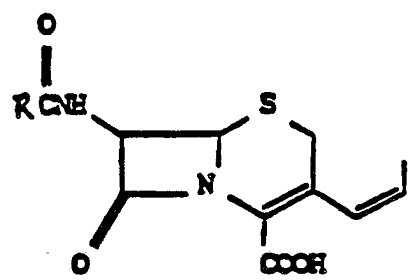

Said vinyl cephalosporines have occupied an important position as oral antibiotics but not as parenteral antibiotics. However, not only do these antibiotics manifest very weak antibiosis to Gram(-) bacteria, as compared to prior parenteral antibiotics, but also their degree of absorption into the body is considerably lower.

Therefore, it was important to develop a broad-spectrum oral antibiotic effective against Gram(-) bacteria which has good absorbability.

The object of this invention is to provide new 3-vinyl cephem derivatives having a substituent in the 10-position which produce broad-spectrum antibiotic action even to Gram(-) bacteria and also have a good degree of absorption into the body.

The present invention relates to new 10-substituted-3-vinyl cephem derivatives of the following general formula I

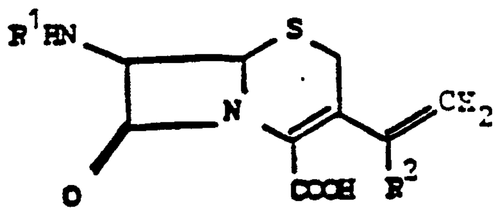

(I)

or a physiologically acceptable salt thereof, wherein $R^1$ is a group of the formula A

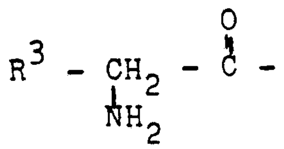

(A)

wherein $R^3$ is phenyl, monosubstituted phenyl or a cyclohexadienyl group of the formula

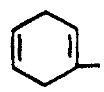

,

or is a group of the formula B

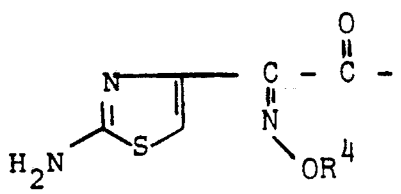

(B)

wherein the $R^4O$-group is in syn-position and $R^4$ denotes $-CH_3$-, $-CH_2COOH$ or $-C(CH_3)_2COOH$ and $R^2$ is $C_1$-$C_4$-alkyl or phenyl.

A preferred definition of $R^2$ as $C_1$-$C_4$-alkyl is methyl, of $R^1$ as monosubstituted phenyl is hydroxy phenyl.

Physiologically acceptable salts are those usually used in pharmaceutical preparations, especially the alkali metal salts, preferably the sodium salt.

The invention furthermore relates to a process for the preparation of compounds of the general formula I and their physiologically acceptable salts, which comprises reacting a compound of the general formula II

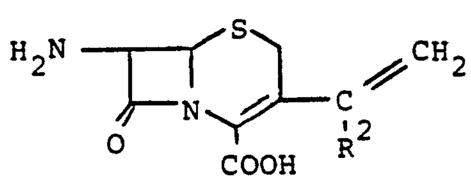

(II)

wherein $R^2$ has the meaning given above and wherein the carboxy group is in protected form, with a reactive derivative of a compound of the general formulae IIIa or IIIb

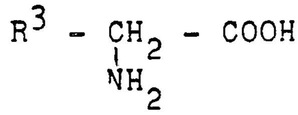 (IIIa)

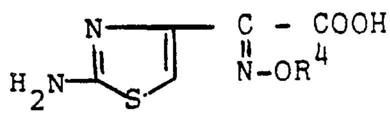 (IIIb)

wherein $R^3$ and $R^4$ have the meaning given above and the $R^4$O-group is in syn-position,
wherein the amino group in formula IIIb may also be protected and
wherein a carboxyl group in $R^4$ carries a protecting group and
splitting off any protective group present in the obtained compound of the formula I and - if desired - converting a carboxylic acid of the formula I into a physiologically acceptable salt.

In the above process the carboxyl and amino groups are protected by protecting groups well-known from the literature describing the snythesis of cephalosporines, as for example ester groups, e.g. the diphenylmethyl ester group. The amino group is preferably used in free from; if protected, usual amino protective groups known from literature can be used, as for example the formyl group.

The activation of a carboxylic acid of the formula IIIa or IIIb is also very well known from literature and may be carried out for example via the carboxylic acid chloride or also by using a carbodiimide.

The acylating reaction is carried out in the usual manner very well known from literature, i.e. in an inert solvent and in a temperature range from about -80 to about +80 °C, preferably between about -20 °C and room temperature.

The protective groups may be split off in a manner well-known from cephalosporine literature, for example by anisol/trifluoroacetic acid.

The formation of physiologically acceptable salts is also carried out in the usual manner known from literature.

Starting compounds of the formulae IIIa and IIIb are well-known or can be prepared according to processes described in literature.

The present invention also relates to the intermediates of the general formula IIa

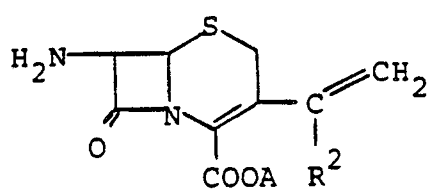 (IIa)

wherein $R^2$ has the meaning given above and A stands for hydrogen or a usual carboxylic acid protective group well known from cephalosporin or peptide chemistry. Intermediates of the formula IIa can for example be obtained as follows:

The 7-position amino group of 7-amino-cephalosporic acid shown by the following formula (1) is protected by a phenylacetyl group, and the double bond in position 3 is transferred to position 2. As a result, 2-cephalosporic acid (hereinafter referred to as "2-cephem") shown by the following formula (3) is formed.

2-cephem, i.e. a cephem having a double bond in position 2, generally shows good reactivity and a high yield in comparison with a cephem having a double bond in position 3, therefore, said processes wherein the 2-cephem of formula(3) is formed are the most important processes of the procedure for the preparation of the subject compound.

Said processes can be represented as follows;

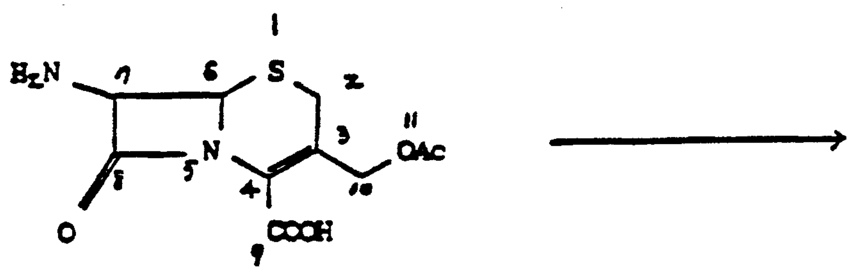

(1)

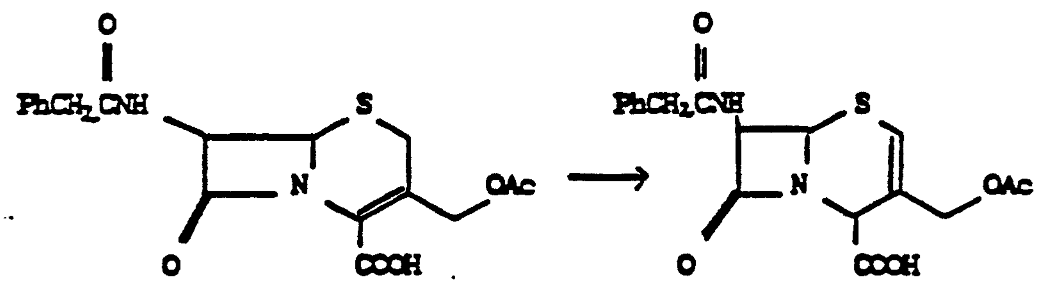

(2) (3)

The resultant 2-cephem of formula(3) is hydrolyzed to the compound of the following formula(4) having a hydroxymethyl group in position 3, and this hydroxymethyl group is selectively oxidized using Jone's reagent (i.e. an aqueous solution of chromium(VI) oxide and sulphuric acid, in the correct stoichiometric proportions) to a formyl group. As a result, the aldehyde compound of the following formula(5) is obtained, continuously, the 4-position carboxyl group of formula(5) is protected as a diphenylmethylester. As a result of this protection, the aldehyde compound of the following formula (6) is obtained.

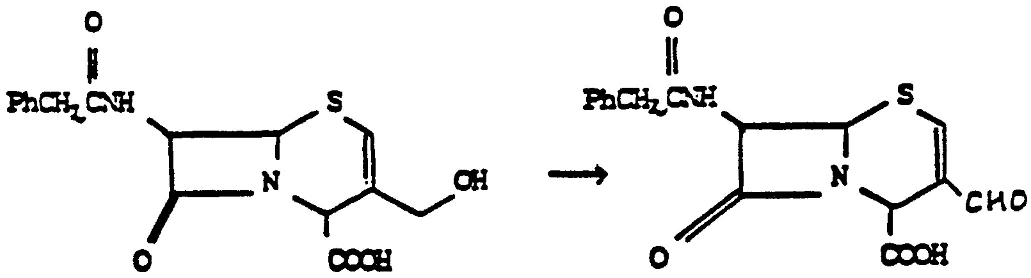

(4) (5)

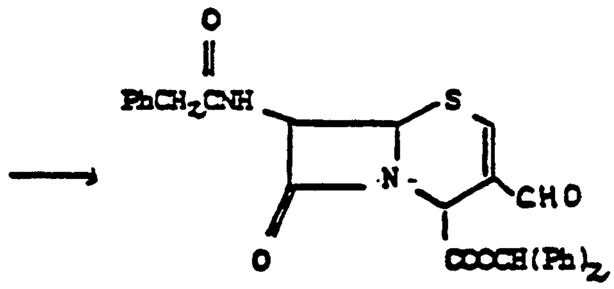

(6)

Also, the aldehyde compound shown by formula(6) can be obtained in accordance with another process. That is, after the acetyl group in position 3 of the above formula (3) is hydrolyzed, the carboxyl group in position 4 is protected by a diphenylmethyl group. Simultaneously with the protection, the hydroxymethyl group in position 3 is oxidized under Jone's conditions. As a result, the aldehyde compound of formula (6) is obtained with a yield similar to that of the former process.

The resultant aldehyde compound is a very important intermediate on the grounds that it can be variously substituted in position 10 by Grignard reagents, RMgX.

For example, the aldehyde compound formula (6) can be reacted with methylmagnesium iodide ($CH_3MgI$) to obtain a secondary alcohol,formula (7) (wherein R = $CH_3$).In this reaction, a stereoisomer with a new stereo-center is produced, and then the resultant compound (7) exhibits two spots with $R_f$ values of 1:1 a TLC plate.

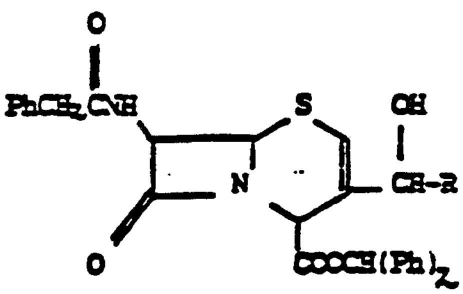

(7)

In the case of 3-cephem, the secondary alcohol formed in the Grignard reaction usually reacts with the ester in position 4 to form a lactone. On the other hand, in the case of 2-cephem, the undesirable formation of a lactone does not occur, and thus, 2-cephem is a desirable intermediate unlike 3-cephem.

In the present invention, the secondary alcohol of formula (7) is oxidized under Jone's conditions to produce a methyl ketone compound of formula (8) with a 55 % yield from the aldehyde compound of formula (6).

In order to change the carbonyl group of the resultant methylketone compound to a methylene group, it was found that the 10-substituted-3-vinyl derivative of the following formula (9) (wherein R = $CH_3$) was prepared by reacting a reagent composed of a $TiCl_4$-Zn-$CH_2Br_2$ complex with the methylketone compound

of formula (8).

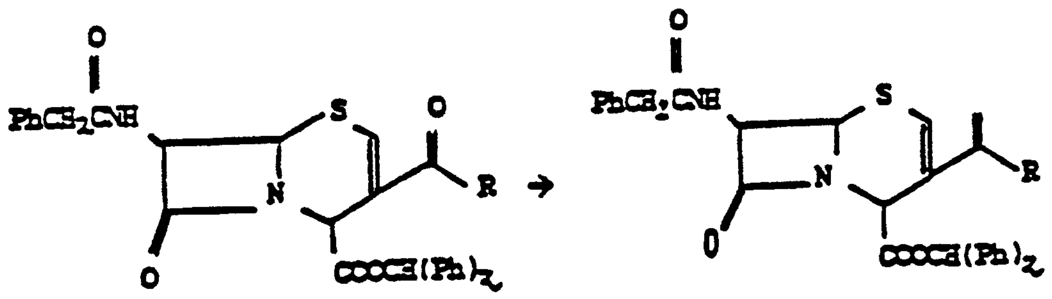

(8) (9)

Subsequently, the double bond in position 2 of formula (9) is transferred to the 3-position and the group protecting the amino group in position 7 is substituted with a reactive functional group and the ester in position 4 is also converted into a carboxyl group. As a result of such processes, the object compound is finally obtained.

To state the above reactions more concretely, the 1-position sulfide in 2-cephem of formula (9) is oxidized with meta-chloroperbenzoic acid to a sulfoxide, as a result the 2-position double bond is transferred to the 3-position as in formula (10) (wherein R = $CH_3$) and said sulfoxide is reacted with phosphorustribromide ($PBr_3$) and is reduced to a sulfide. Accordingly, the 3-cephem of formula (11) (wherein R = $CH_3$) is prepared.

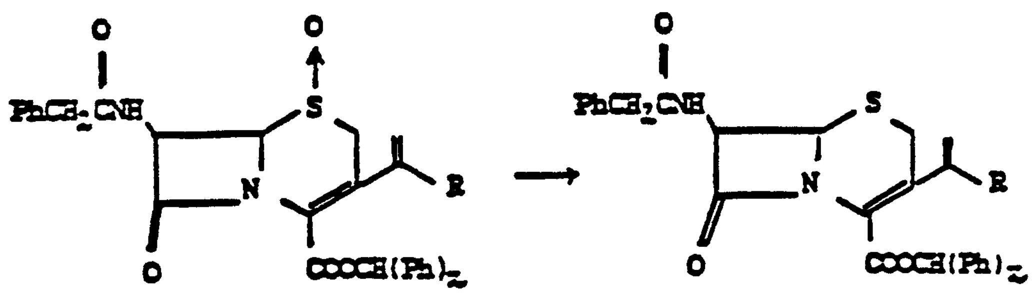

(10) (11)

Thereafter, the phenylacetyl blocking group in the 7 position of 3-cephem is removed using phosphorus penta chloride ($PCl_5$), and then 7-amino-3-cephem of the following formula (12) (wherein R = $CH_3$) is obtained.

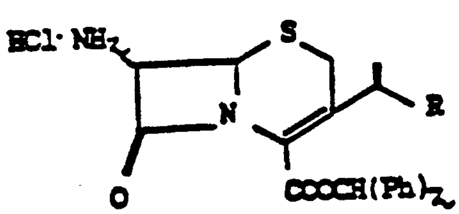

(12)

If desired, this compound can easily be transformed into the free carboxylic acids by usual methods described in literature.

The 7-amino-3-cephem of formula (12) so formed which is one of the possible starting materials of the formula II can be converted into various derivatives of 10-substituted-3-vinyl cephalosporines depending upon which functional groups are used as substituents. For example, if compound (12) is reacted with an aminothiazole derivative of the following formula (C) using Vilsmeyer reagent, as a result, the object

compound of formula(13)-(wherein R represents said formula (C) ) is prepared, which is substituted in position 7 with the compound of formula (C).

When the groups protecting the amino groups and the carboxyl group in the compound of formula (13) are removed, a new oral antibiotic according to the general formula I is synthesized.

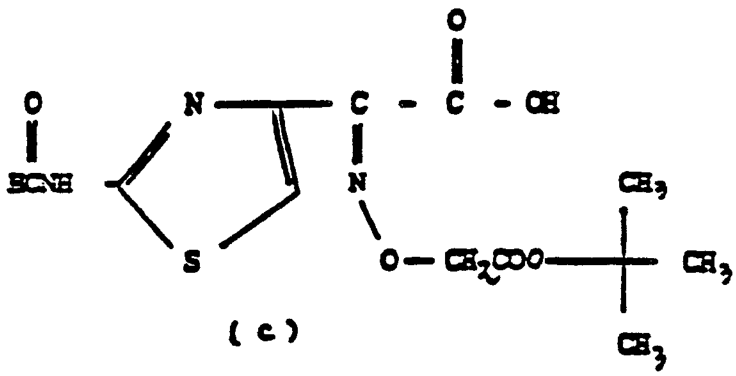

( c )

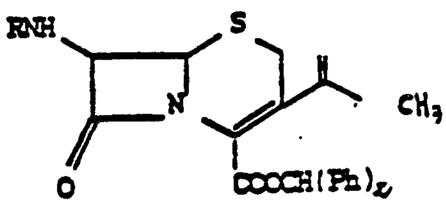

(13)

The invention also relates to pharmaceutical preparations for the treatment of microbial infections, which contain one or more of the compounds of the general formula I or their physiologically acceptable salts.

The pharmaceutical preparations according to the invention can also be used in combinations with other active compounds, for example from the series comprising penicillins, cephalosporins and aminoglycosides.

The compounds of the general formula I and their physiologically acceptable acid addition salts can be administered orally, intramuscularly or intravenously, preferably orally.

Pharmaceutical preparations which contain one or more compounds of the generla formula I or their salts, as the active substance can be prepared by a process in which the compounds of the formula I or their salts are mixed with several pharmacologically acceptable excipients or diluents, such as, for example, fillers, emulsifiers, lubricants, flavor correctants, colorants or buffer substances, and the mixture is brought into a suitable galenical formulation form, such as, for example, tablets, coated tablets, capsules or a suspension or solution suitable for parenteral administration.

Examples of excipients or diluents which may be mentioned are tragacanth, lactose, talc, agar-agar, polyglycols, ethanol and water. Buffer substances are, for example, organic compounds, such as, for example, N,N′-dibenzylethylenediamine, diethanolamine, ethylenediamine, N-methylglucamine, N-benzylphenethylamine, diethylamine and tris-(hydroxymethyl)aminomethane, or inorganic compounds, such as, for example, phosphate buffer, sodium bicarbonate or sodium carbonate. Suspensions or solutions in water with or without buffer substances are preferably suitable for parenteral administration. It is also possible to administer the active compounds as such in a suitable form, for example in capsules, without an excipient or diluent.

Suitable doses of the compounds of the general formula I or their physiologically acceptable salts are about 0.2 to 5 g/day, preferably 0.5 to 3 g/day, for an adult with a body weight of about 60 kg.

Individual or in general multiple doses can be adminsitered, it being possible for the individual dose to contain the active compound in an amount of about 50 to 1,000 mg, preferably about 100 to 500 mg.

This invention is illustrated by the following Examples, but should not be construed to be limited thereto.

The following Examples 1 to 12 describe the manufacture of a starting material.

Example 1; Preparation of 7β-phenylacetamido-3-acetoxymethyl-3-cephem-4-carboxylic acid

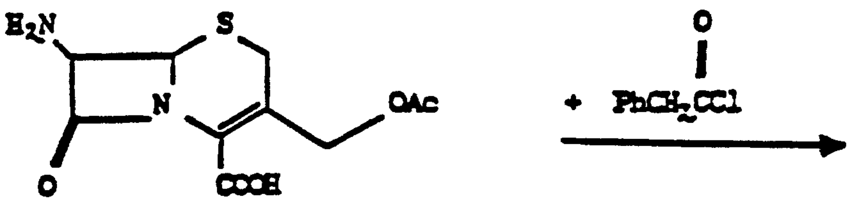

,

(1)

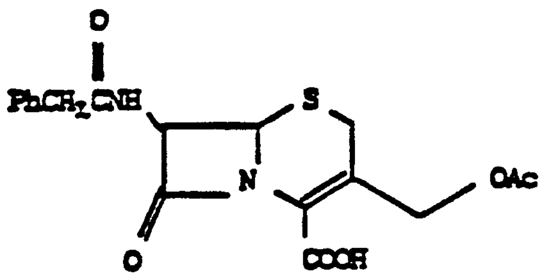

(2)

165g of 7-ACA (0.6 mole) are suspended in 1.8 l of water and 1.4 l of acetone and 165 g of sodium hydrogen carbonate is slowly added while stirring. The solution is cooled to 5°C and after 108 g of phenylacetylchloride (0.7 mole) dissolved in 450 ml of acetone has been slowly added, the solution is again stirred at 5°C for 1 hr. The solution is adjusted to pH 2 with conc. HCl and then extracted with 1500 ml ethylacetate.

The extracts are dried with anhydrous sodium sulfate and the solvent is distilled under reduced pressure to obtain 210g of 7β-phenylacetamino cephalosporic acid (yield; 90%).

NMR(DMSO-$d^6$)δ : 2.00(s,3H,$CH_3$), 3.45(ABq,2H,C-2)
3.50(s,2H,$PhCH_2$), 4.85(ABq,2H,$CH_2OAc$)
4.95(d,1H,C-6), 5.65(dd,1H,C-7)
7.30(s,5H,Ph), 9.05(d,1H,NH)

Example 2; Preparation of 7β -phenylacetamido-3-acetoxymethyl-2-cephem-4-carboxylic acid

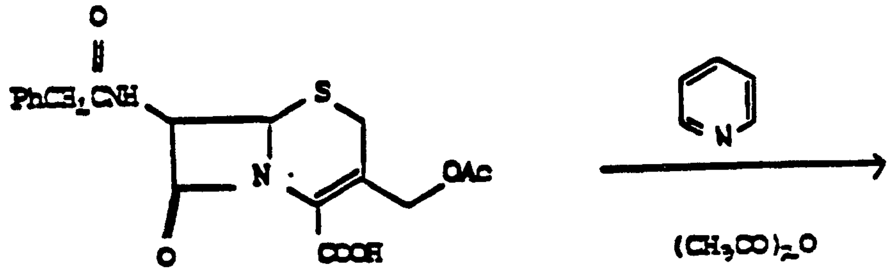


(2)

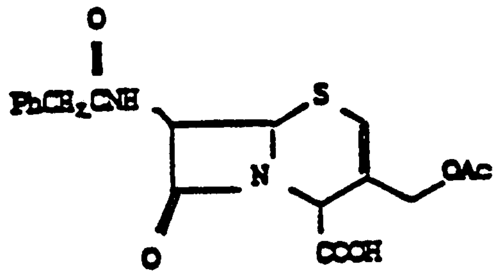

(3)

110g of 7β -phenylacetamido-3-acetoxymethyl-3-cephem-4-carboxylic acid obtained by Example 1 are dissolved in 500 ml of pyridine and cooled to 0° C, then 42 ml of acetic acid anhydride is slowly added while stirring. The solution turns black and a solid is produced.

The reaction mixture is maintained at 0° C for 20 hrs and then a mixture of 200 ml of ethylacetate and 400 ml of ethylether is added and the solid products are filtered.

The resultant white solid is added to a mixture of 1 l of water and 2 l of ethylacetate. 100 ml of 2N HCl is added with stirring and the organic layer is separated, washed with brine, and dried using anhydrous magnesium sulphate. The solvent is distilled under reduced pressure to obtain 60g of 7β -phenylacetamido-3-acetoxymethyl-2-cephem-4-carboxylic acid, formula (3)(yield; 54.5%).

NMR(DMSO-$d^6$)δ ;2.00(s,3H,$CH_3$), 3.50(s,2H,$PhCH_2$)
4.65(s,2H,$PhCH_2OAc$), 4.85(s, 1H,C-4)
5.15(d,1H,C-6),5.90(dd,1H,C-7)
6.60(s,1H,C-2),7.30(s,15H,Ph)
9.15(d,1H,NH)

Example 3; Preparation of diphenylmethyl 7β -phenyl acetamido-3-hydroxymethyl-2-cephem-4-carboxylate

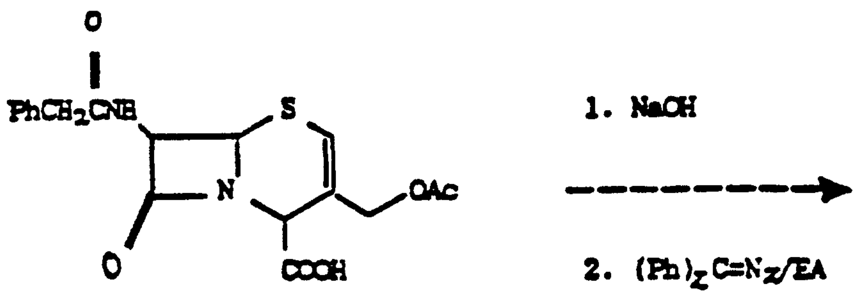


(3)

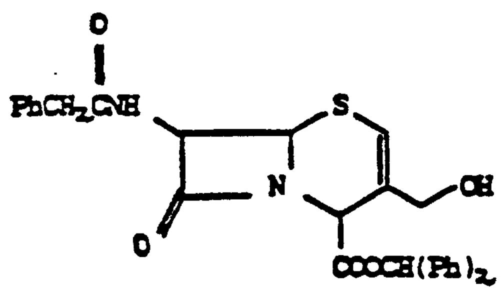

(4)'

15g of 7β -phenylacetamido-3-acetoxymethyl-2-cephem-4-carboxylic acid(0.028mole) obtained by Example 2 are dissolved in a mixture of 200 ml of water, 20 ml of acetone and 80 ml of 1N NaOH and stirred at 50° C for 24hrs.

The mixture is cooled to room temperature, adjusted to pH 2 with 2N HCl and extracted with 500 ml of ethylacetate.

15g diphenylmethyl diazomethane is added to the solution and the resultant solution is stirred for 3 hrs.

The solvent is distilled under reduced pressure and the remains are washed with isopropyl ether to obtain 15g of white solid diphenylmethyl 7β -phenylacetamido-3-hydroxymethyl-2-cephem-4-carboxylate of formula (4)' (yield; 76%).

---NMR($CDCl_3$) : 3.60(s,2H,$PhCH_2$), 4.08(s,2H,$CH_2OH$)
4.15(s,1H,$CH_2OH$), 5.15(d,1H,C-6)
5.20(s,1H,C-4), 5.50(dd,1H,C-7)
6.30(s,1H,C-2), 6.80(s,1H,$CHPh_2$)
7.30(s,15H,Ph), 7.80(d,1H,NH)

Example 4; Preparation of diphenylmethyl 7β -phenylacetamido-3-formyl-2-cephem-4-carboxylate

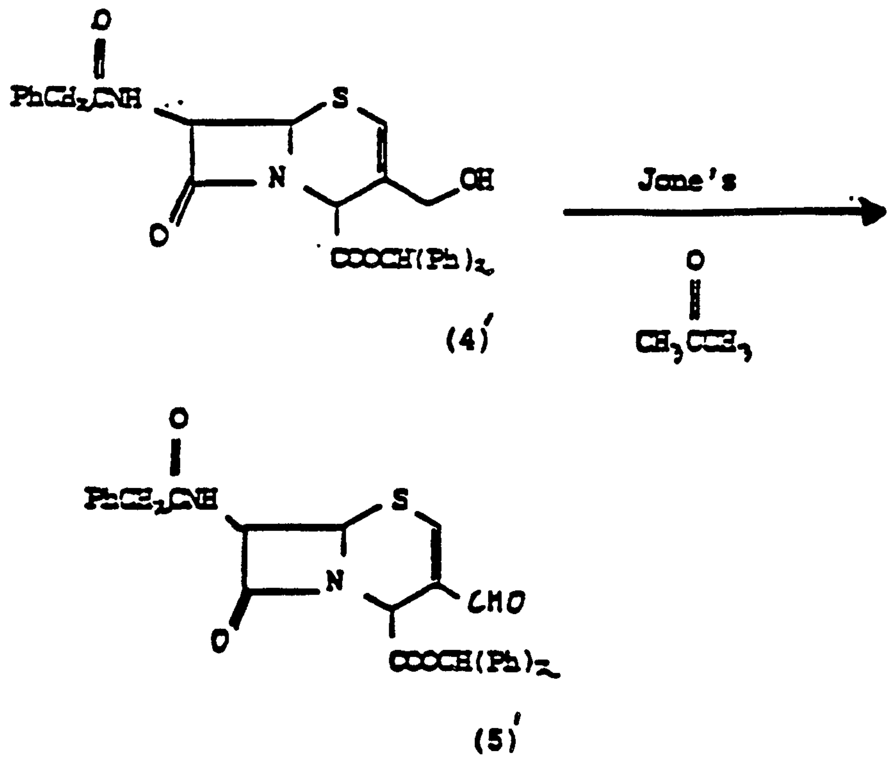


(4)'

(5)'

15g of diphenylmethyl 7β -phenylacetamido-3-hydroxymethyl-2-cephem-4-carboxylate (29mmole) obtained by Example 3 are dissolved in 500 ml of acetone and Jone's reagent, prepared from chromium trioxide and sulfuric acid, and then water is slowly added until the color of the reaction mixture becomes red.

The mixture is stirred again for 5 mins and isopropylalcohol is added. The solution is stirred for 5 mins more until it turns green, and then the solvent is distilled under reduced pressure. The organic layer is separated by adding 300 ml of ethylacetate and 300 ml of water and washed in the order of water, an aqueous solution of sodium hydrogen carbonate, water, 1N HCl and brine. The remains are dried with anhydrous magnesium sulfate and the solvent is distilled under reduced pressure.

Chromatcgraphy is performed with a mixed solution of toluene and ethylacetate using Silica gel to obtain 6g of solid, white diphenylmethyl 7β -phenylacetamido-3-formyl-2-cephem-4-carboxylate of formula (5)' (yield; 40%).

NMR($CDCl_3$); 3.60(s,2H,$PhCH_2$), 5.10(d,1H,C-6)
5.40(dd,1H,C-7), 5.45(1H,s,C-4)
6.50(d,1H,NH), 6.80(s,1H,$CHPh_2$)
7.30(s,15H,Ph), 7.40(s,1H,C-2)
9.30(s,1H,CHO)

Example 5 ; Preparation of 7$\beta$ -phenylacetamido-3-hydroxymethyl-2-cephem-4-carboxylic acid

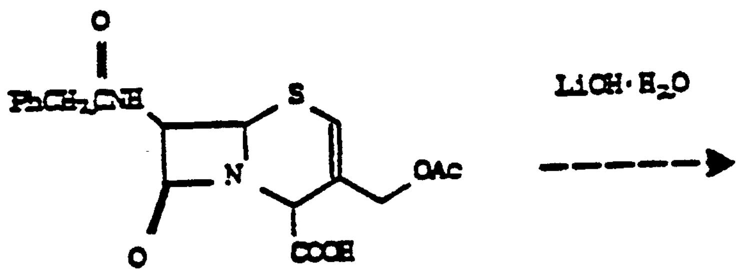


(3)

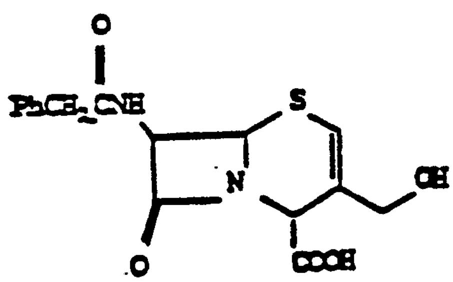

(4)

34g of 7$\beta$ -phenylacetamido-3-acetoxymethyl-2-cephem-4-carboxylic acid obtained as in Example 2 are dissolved in a mixture of 1,000 ml of water and 9g of lithium hydroxide, stirred at 20° C for 2 hrs and adjusted to pH 2 by adding 2N HCl. The solution is extracted with 500 ml of ethylacetate, washed with brine and dried with anhydrous magnesium sulfate. The solvent is distilled under reduced pressure and eliminated to obtain 7$\beta$-phenylacetamido-3-hydroxymethyl-2-cephem-4-carboxylic acid of formula (4).

Example 6 ; Preparation of 7β -phenylacetamido-3-formyl-2-cephem-4-carboxylic acid

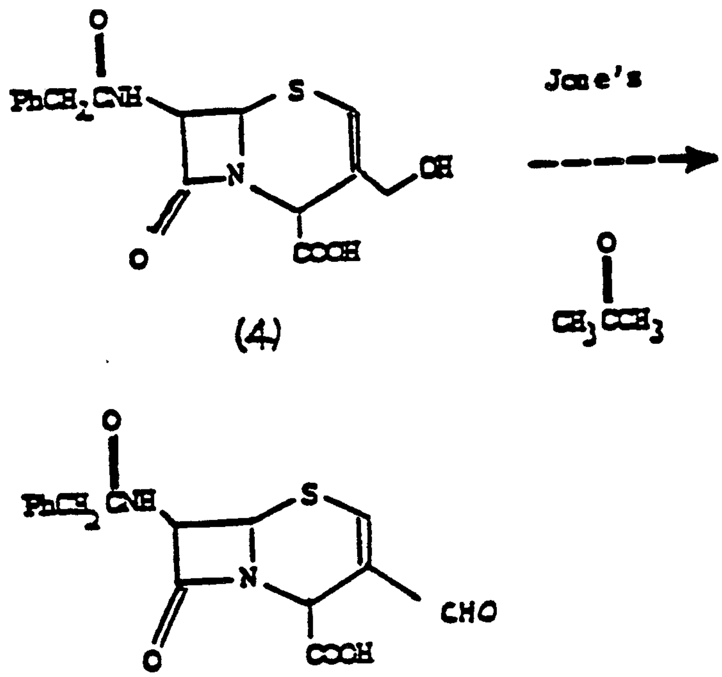


(4)

(5)

15g of 7β -phenylacetamido-3-hydroxymethyl-2-cephem-4-carboxylic acid obtained by Example 5 are dissolved in 200 ml of acetone. Jone's reagent is added to the solution and when the color of the solution becomes red, isopropyl alcohol is added to change color of the solution to green.

The solvent is distilled under reduced pressure and the remains are dissolved in 500 ml of ethylacetate,washed with brine and dried using anhydrous magnesuim sulfate. The solvent is distilled under reduced pressure and removed to obtain 7β -phenylacetamido-3-formyl-2-cephem-4-carboxylic acid of formula (5) (yield; 70%).

Example 7; Preparation of diphenylmethyl 7β -phenylacetamido-3-(1-hydroxyethyl)-2-cephem-4-carboxylate

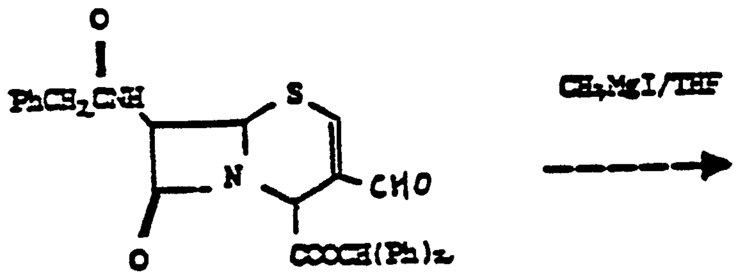


(6)

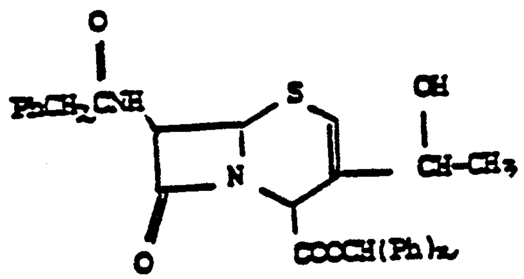

(7)

6g of diphenylmethyl 7β -phenylacetamido-3-formyl-2-cephem-4-carboxylate (11mmole) obtained as in Example 4 are dissolved in 100 ml of THF and cooled to - 78° C. 4 equivalents of methyl magnesium iodide are added. The solution is strongly stirred for 30 mins, adjusted to pH 2 by adding 1N HCl and warmed from - 78° C to room temperature. The solution is extracted with 100 ml of ethyl acetate, washed with brine and dried using magnesium sulfate. The solvent is distilled under reduced pressure.

Chromatography is performed with a mixed solution of toluene and ethylacetate using Silica gel to obtain 4g of white, solid diphenylmethyl 7β -phenylacetamido-3-(1-hydroxyethyl)-2-cephem-4-carboxylate of formula (7) (yield;65%).

NMR($CDCl_3$) ; 1.17 (d, 3H, $Ch_3$), 1.80 (s, 1H, OH),
3.60 (s, 2H, $PhCH_2$), 4.95 (s, 1H, C-4),
5.10 (d, 1H,C-6), 5.50 (dd,1H, C-7)
6.25 (s, 1H,C-2), 6.30 (d, 1H, NH),
6.80 (s, 1H, $CHPh_2$), 7.30 (s, 15H, Ph).

Example 8 : Preparation of diphenylmethyl 7β -phenylacetamido-3-acetyl-2-cephem-4-carboxylate

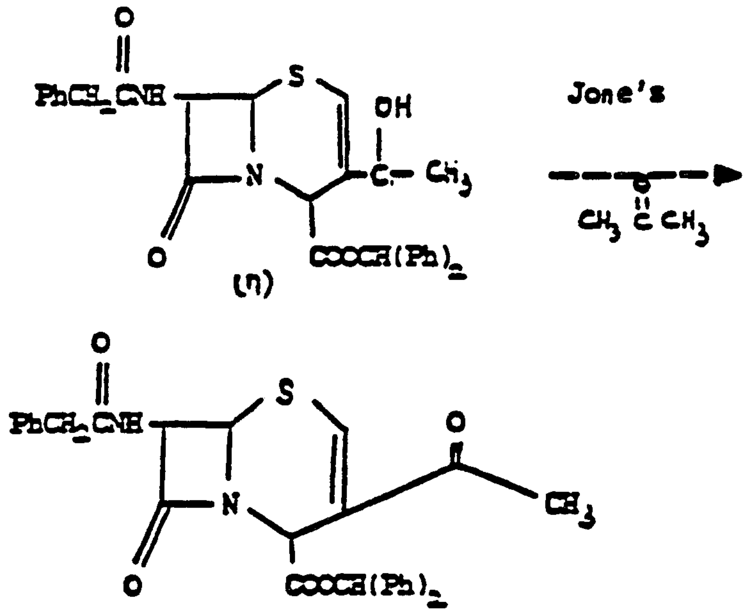


(7)

(8)

4g of diphenylmethyl 7β -phenylacetamido-3-(1-hydroxyethyl-2-cephem-4-carboxylate(7mmole) obtained by Example 7 are dissolved in 100ml of acetone.
The reaction mixture turns red following the slow addition of Jone's reagent, it is stirred for an additional 5 mins and then isopropyl alcohol is added until the color of the solution becomes green. After 10 mins, the solvent is distilled under reduced pressure and the organic layer is separated by adding ethylacetate and water, washed with the following solutions in this order: water,an aqueous solution of sodium hydrogen carbonate, water, 1N HCl and brine and then dried using anhydrous sodium sulfate. The solvent is distilled under reduced pressure.

Chromatography is performed with a solution mixture of toluene and ethyl acetate using Silica gel to obtain 2g of solid, yellow diphenyl methyl 7β -phenylacetamido-3-acetyl-2-cephem-4-carboxylate of formula(8) - (yield : 50%).

NMR($CDCl_3$) ; 2.18 (3H, S,

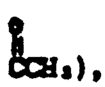

3.58 (2H, s, $PhCH_2$),
4.80 (1H, d, C-6), 5.30 (1H, dd, C-7),
5.50 (1H, s, C-4), 6.45 (1, d, NH),
6.50 (1H, s, $Ph_2CH$), 7.30 (15H, s, Ph),
7.40 (1H, s, C-2)

Example 9 : preparation of diphenylmethyl 7β -phenylacetamido-3-isopropenyl-2-cephem-4-caboxylate

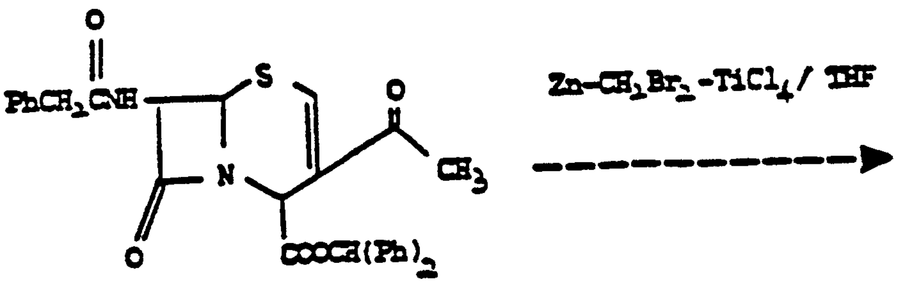


(8)

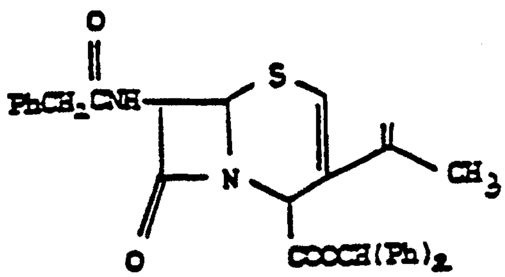

(9)

17.9g of zinc powder are added to a mixture of 6.28ml (90mmole) of dibromomethane and 160ml of THF. The solution is stirred well at -40° C and 7.15ml of $TiCl_4$ is slowly added to the solution. The solution is continuously stirred at 0° C for 10 hrs.

10g of diphenylmethyl 7β -phenylacetoamido -3- acetyl -2-cephem-4-carboxylate(18mmole) obtained as in Example 8 are added to the above reaction mixture and after the solution has been stirred at 5° C for 3 hrs, 1N HCl is slowly added.

200ml of ethylacetate are added to the solution and 200ml of 1N HCl are further added. The organic layer is seperated, washed with 1N HCl and brine and dried with anhydrous magnesium sulfate. The solvent is distilled under reduced pressure.

Chromatography is performed with a solution mixture of tolune and ethylacetate using Silica gel to obtain 8.0g of diphenylmethyl 7β -phenylacetamido-3-isopropenyl-2-cephem-4-carboxylate of formula(9) (yield : 80%).

NMR($CDCl_3$) ; 1.80 (s, 3H, $CH_3$), 3.60 (s, 2H, $PhCH_2$),
4.80 (d, 2H, $CH_3C=CH_2$), 5.10 (d, 1H, C-6),
5.30 (s, 1H, C-4), 5.50 (dd, 1H, C-7),
6.30 (d, 1H, NH), 6.40 (s, 1H, C-2),
6.80 (s, 1H, $CHPh_2$), 7.30 (s, 15H, Ph).

Example 10 ; Preparation of diphenylmethyl 7$\beta$ -phenylacetamido-3-isoprophenyl-3-cephem-4-carboxylate-1-oxide

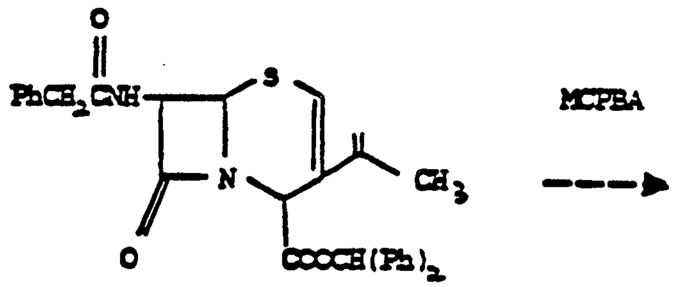


(9)

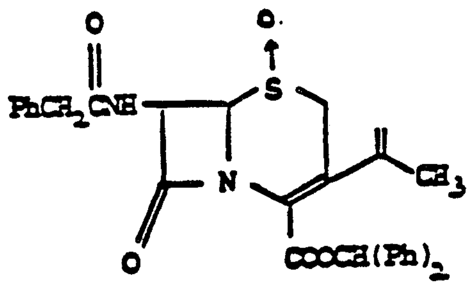

(10)

8g of diphenylmethyl 7$\beta$ -phenylacetamido-3-isopropenyl-2-cephem-4-carboxylate(15mmole) obtained as in Example 9 are dissolved in 200ml of dichloromethane.

The solution is cooled to 5° C. 5.16g of 3-chloroperoxy benzoic acid is added to the solution and it is stirred for 1 hr.

After the solution has been washed with a saturated aqueous solution of sodium hydrogen carbonate and brine, it is dried using anhydrous magnesuim sulfate. The solvent is distilled under reduced pressure and eliminated.

Chromatography is performed with a solution mixture of tolune and ethylacetate using Silica gel to obtain 4.2g of diphenylmethyl 7$\beta$ -phenylacetamido-3-isopropenyl-3-cephem-4- carboxylate-1-oxide of formula(10) (yield ; 50%).

NMR($CDCl_3$) ; 1.60 (s, 3H, $CH_3$), 3.60 (s, 2H, $PhCH_2$),
3.65 (ABq, 2H, C-2), 4.40 (d, 1H, C-6),
4.75 (s, 2H, $CH_3$-C=$CH_2$), 6.60 (dd, 1H, C-7),
6.70 (d, 1H, NH), 6.90 (s, 1H, $CHPh_2$),
7.30 (s, 15H,Ph).

Example 11 : Preparation of diphenylmethyl 7β -phenylacetamido-3-isopropenyl-3-cephem-4-carboxylate

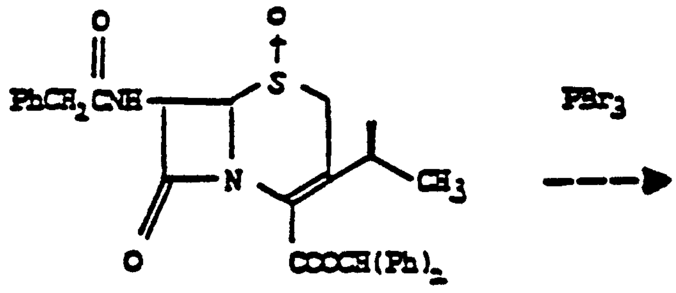


(10)

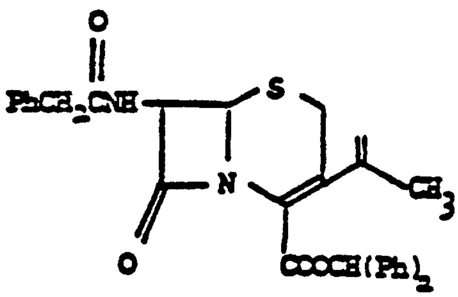

(11)

4.0g(7.4mmole) of diphenylmethyl 7β -phenylacetamido-3-isopropenyl-3-cephem-4-carboxylate obtained from the above Example 10 are dissolved in 200 ml of TMF and cooled to -78°C, then 3.00g-(11mmole) of $PBr_3$ are added while stirring for 1 hour.

200ml of ice water is added to the solution, and the resulting solution is extracted with 200ml of ethylacetate. The organic layer is washed with salt-water, and dried using magnesium sulfate anhydride and then the solution is distillated under reduced pressure in order to eliminate the solvent.

Finally, the resulting solution is chromatographed with toluene and ethylacetate using silica gel, consequently, 2.3g(55%) of diphenylmethyl 7β -phenylacetamido-3-isopropenl-3-cephem-4-carboxylate represented by formula(11) is obtained.

NMR($CDCl_3$) ; 1.70(s, 3H, $CH_3$), 3.33(s, 2H, C-2),
3.60(s, 2H, $PhCH_2$), 4.65(s, 2H, $CH_3C=CH_2$),
5.95(d, 1H, C-6), 5.75(dd, 1H, C-7),
6.20(d,1H, NH), 6.90(s, 1H, $CHPh_2$),
7.30(s, 15H, Ph).

Example 12 : Preparation of diphenylmethyl 7-amino-3-isopropenyl-3-cephem-4-carboxylate hydrochloride

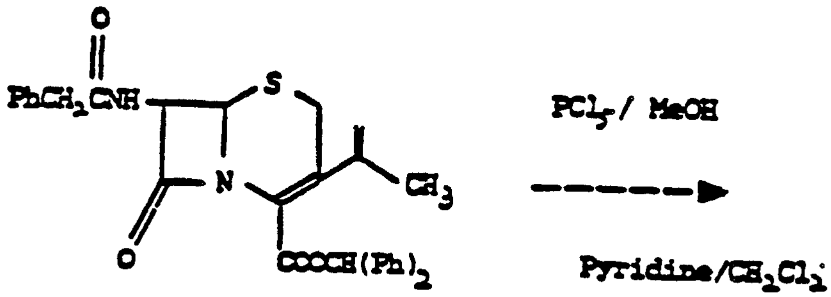


(11)

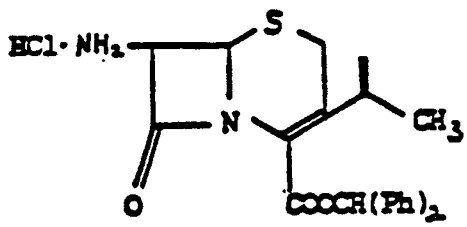

(12)

1.04g(5mmole) of $PCl_5$ is suspended in 15ml of dichloromethane and cooled to 5° C. 0.403g(5mmole) of pyridine is added to the suspension and then stirred for 1 hour, then 140mg(0.27mmole) of diphenylmethyl 7$\beta$ -phenylacetamido-3-isopropenyl-3-cephem-4-carboxylate is added at once and stirred for 1 hour at the same temperature.

Subsequently, the temperature of the solution is lowered to -40° C, 10ml of methyl alcohol is added therein, and it is stirred for 2 hours at a temperature below -10° C. The solution is distilled under reduced pressure in order to eliminate the solvent.

Water and ethylether are added therein, the resulting solid is seperated from the solution. Consequently, 87mg(80%) of diphenylmethyl 7-amino-3-isopropenyl-3-cephem-4-carboxylate hydrochloride represented by formula (12) is obtained.

The following Examples describe the manufacture of compounds covered by the general formula I. "Z" is known from literature as corresponding to "syn".

Example 13 : Preparation of diphenylmethyl 7β-[(Z)-2-(2-formyl amido-4-thiazol)-2-(t-butoxycarbonylmethoxyimino)-acetamido]-3-isopropenyl-3-cephem-4-carboxylate

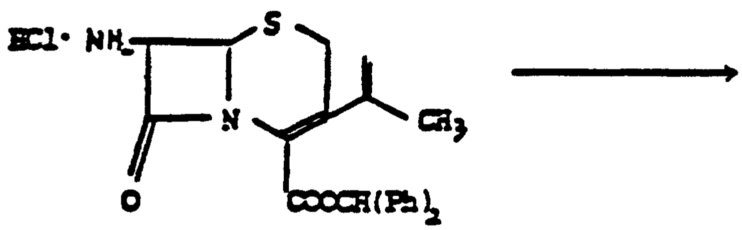

(12)

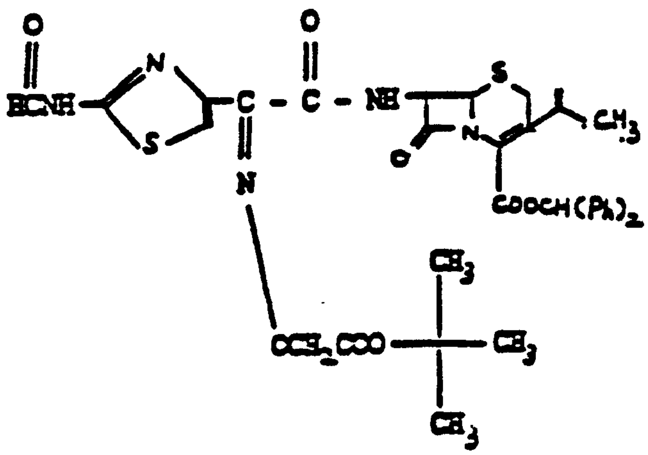

(13)

A mixture of DMF 0.04ml(5mmole) and THF 2ml is cooled to -5° C, and 0.05ml(5mmole) of $P(O)Cl_3$ is added therein. The mixture is stirred for 30 minutes, and 130mg(4mmole) of 2-(2-formylamido-4-thiazol)-2-(t-butoxycarbonylmethoxyimino)-acetic acid is added therein, and the resulting solution (A) is stirred at -5° C for 1 hour.

50mg(0.113mmole) of diphenylmethyl 7-amino-3-isopropenyl-3-cephem-4-carboxylate hydrochloride is suspended in 4ml of ethylacetate, 0.8ml(30mmole)of BSA is added therein and the resulting solution (B) is cooled to -20° C.

Solutions (A) and (B) are mixed and stirred at -20° C for 1 hour. Accordingly, water and ethylacetate are added to the mixture, the organic layer is separated, and the mixture is washed with a 10% aqueous solution of sodium hydrogen carbonate and salt-water and dried using magnesium sulfate auhydride. The solvent is eliminated.

Consequently, 80mg(90%) of diphenylmethyl 7β -[(Z)-2-(2-formylamido-4-thiazol)-2-(t-butoxycarbonylmethoxyimino)-acetamido]-3-isopropenyl-3-cephem-4-carboxylate, a yellow colored solid, represented by formula (13) is obtained.

NMR($CDCl_3$) : 1.30(s, 9H, t-Bu), 1.63(s, 3H, $CH_3$-C = $CH_2$),
3.40(s, 2H, C-2), 4.55(s, 4H, $OCH_2$ and
$CH_2$ = C-$CH_3$), 5.00(d, 1H C-6),
5.83(dd, 1H, C-7), 6.65(s, 1H, $CHPh_2$),
7.30(s, 15H, $Ph_2$), 7.30(s, 1H, ATMO),

O O
|| ||
8.48(s, 1H, HCNH), 8.85(d, 1H, NH-C),

O
||
12.20(s, 1H, HCNH)

Example 14 : Preparation of diphenylmethyl 7β -[(Z)-2-(2-amino-4-thiazol)-2-(t-butoxycarbonylmethoxyimino) acetamido]-3-isopropenyl-4-carboxylate

80mg of the compound from the above Example 13 is dissolved in 10ml of methanol, therein 1ml of concentrated HCl is added. The resulting mixture is stirred for 1 hour and neutralized with a 5% aquoeus solution of sodium hydrogen carbonate.

After that, the solution is distilled to eliminate the solvent, extracted with ethylacetate, washed with an aqueous solution of sodium hydrogen carbonate and salt-water, dried using magnesium sulfate anhydride and again distilled to eliminate the solvent.

Finally, the solution is chromatographed using silica gel. Consequently, 50mg of diphenylmethyl 7β -[-(Z)-2-(2-amino-4-thiazol)-2-(t-butoxycarbonylmetoxyimino) acetamido-3-isopropenyl-4-carboxylate is obtained.

Example 15 : Preparation of 7β -[(z)-2-(2-amino-4-thiazol)-2-(carboxy-methoxy-imino)acetamido]-3-isopropenyl-4-carboxylic acid

The compound obtained from the above Example 14 is dissolved in 20ml of dichloromethane, 1ml of anisol and 1ml of trifluoroacetic acid are added therein at 0° C. The mixture is stirred at the same temperature for 1 hour and at room temperature for 2 hours, then the solvent is eliminated by distillation under reduced pressure. Finally, isopropylether is added and 30mg of the resulting yellow colored solid is seperated.

NMR(DMSO-$d^6$) : 1.85(s, 3H, $CH_3$-C = $CH_2$), 3.50 (ABq, 2H, C-2),
4.55 (s, 2H,

O
||
$OCH_2COH$),

4.80 (d, 2H, $CH_2$ = C = $CH_3$),
5.15 (d, 1H, C-6), 5.75 (dd, 1H, C-7),
6.75 (d, 1H, proton of aminothiazol),
9.50 (s, 1H, NH).

Example 16 : Preparation of diphenylmethyl 7β-[(z)-2-(2-amino-4-thiazol-2-(-1-t-butoxycarbonyl-1-methyl)-ethoxyimino-acetamido]-3-isopropenyl-3-cephem-4-carboxylate

$P(O)Cl_3$ (0.05ml) is added to a mixture of THF(2ml) and DMF(0.04ml) which is cooled to -5° C, it is stirred for 30minutes, and then 130mg of 2-(2-amino-4-thiazol)-2-(-1-t-butoxycarbonyl-1-methyl)-ethoxyiminoacetic acid are added therein and stirred at -5° C for 1hour(solution A).

50mg of the compound obtained from Example 12 is suspended in 42ml of ethylacetate and 1ml of bis-trimethyl-silyl-acetamid (BSA) is added therein and cooled to -20° C (solution B).

The previously prepared solution A is slowly added to said solution B at the same temperature as

above and stirred for 1 hour, the organic layer due to the addition of water and ethylacetate is separated.

It is washed with a 10% aquoeus solution of sodium hydrogen carbonate and salt-water, and dried using magnesium sulfate anhydride, after that the solvent is eliminated from it. Consequently, 80mg of diphenylmethyl 7$\beta$ -[(z)-2-(2-amino-4-thiazol)-2-(-1-t-butoxycarbonyl-1-methyl)-ethoxyiminoacetamido]-3-isopropenyl-3-cephem-4-carboxylate is obtained by means of chromatography using silica gel.

NMR($CDCl_3$) : 1.40(s, 9H, t-buthyl), 1.60 (s, 6H, $(CH_3)_2$),
3.65 (ABq, 2H, C-2), 4.80 (d, 2H, $CH_2=C-CH_3$),
5.15 (d, 1H, C-6), 6.05 (dd, 1H, C-7),
6.90 (s, 1H, proton of aminothiazol),
8.70 (s, 2H, $NH_2$), 8.90 (d, 1H, $NH\overset{O}{\overset{\|}{C}}$ )

Example 17 : Preparation of 7$\beta$ -[(z)-2-(2-amino-4-thiazol)-2-(1-carboxy-1-methyl)-ethoxyiminoacetamido]-3-isopropenyl-3-cephem-4-carboxylic acid.

80mg of the compound obtained from Example 12 are dissolved in dichloromethane 20ml, anisol 1ml and trifluoroacetic acid 1ml are added therein.

The solution is stirred at room temperature for 3 hours, and the solvent is eliminated. Isopropylether is added and the resultant solid is removed. Consequently, 45mg of 7$\beta$ -[(z)-2-(2-amino-4-thiazol)-2-(1-carboxy-1-methyl)-ethoxy iminoacetamido]-3-isopropenyl-3-cephem-4-carboxylic acid are obtained.

NMR(DMSO-$d^6$) : 1.40(s, 6H, $(CH_3)_2$), 1.80 (s, 3H, $CH_3-C=CH_2$),
3.50 (ABq, 2H, C-2), 4.70 (d, 2H, $CH_2=C-CH_3$),
5.20 (d, 1H, C-6), 5.70 (dd, 1H, C-7),
6.60 (s, 1H, proton of aminothiazol),
9.60 (d, 1H, NHCO).

Example 18 : Preparation of diphenylmethyl-7$\beta$-[(Z)-2-(2-amino-4-thiazol)-2-methoxyimino-acetamido]-3-isopropenyl-3-cephem-4-carboxylate

A mixture of (z)-2-(2-amino-4-thiazol)-2-(methoxyimino)acetic acid 0.011mole and $POCl_3$ 0.012mole is added to 40ml of ethylacetate, the resultant solution is stirred at 5°C for 30 minutes. Soon after, $POCl_3$ 0.012 mole is added to the mixture and stirred for 30minutes, and then DMF 0.012mole is added and stirred for 1 hour (solution A).

The compound obtained from said Example 12 and bis-trimethyl-silyl-acetamid (BSA) 0.06 mole are added to ethylacetate, and this solution is cooled to -10°C (solution B).

Said solution A is slowly added to said solution B, and the resultant solution is stirred at -10°C for 1 hour, while water and ethylacetate are added.

The extracted organic layer is washed with an aqueous solution of sodium hydrogen carbonate and brine, and dried with magnesium sulfate anhydride. Finally, the solvent is eliminated and the resul ting product is treated with ethylether. Consequently, diphenylmethyl-7$\beta$-[(Z)-2-(2-amino-4-thiazol)-2-methoxyiminoacetamido]-3-isopropenyl-3-cephem-4-carboylate, which is a solid, is obtained.

Example 19 : Preparation of 7$\beta$-[(Z)-2-(2-amino-4-thiazol)-2-methoxyiminoacetamido]-3-isopropenyl-3-cephem-4-carboxylic acid

0.005mole of the compound obtained from Example 18 is dissolved in 20ml of dichloromethane while maintaining a temperature of 5°C, 0.1mole of trifluoroacetic acid and 0.02 mole of anisol are added therein and the resulting solution is stirred at room temperature for 3 hours. The solvent is eliminated by means of distillation, and the solid formed by adding ethylether is separated. Consequently, 7$\beta$-[(Z)-2-(2-amino-4-thiazol)-2-methoxy-iminoacetamido]-3-isopropenyl-3-cephem-4-carboxylic acid is prepared.

Example 20 : Preparation of diphenymethyl 7-cyclohexadienylaminoacetamido-3-isopropenyl-3-cephem-4-carboxylate

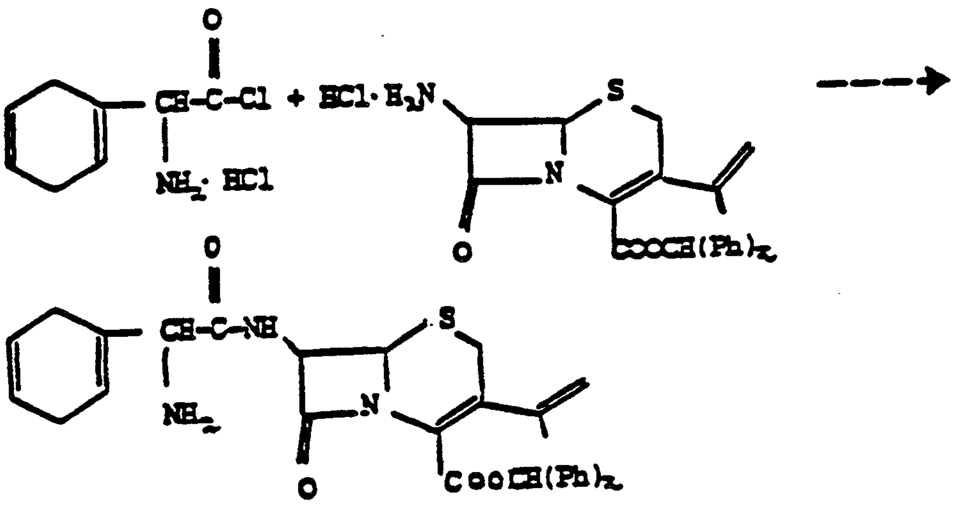

155mg of diphenylmethyl 7-amino-3-isopropenyl-3-cephem-4-carboxylate hydrochloride obtained from Example 12 are dissolved in N,N-dimethylformamide 2.0ml and the solution is cooled to 0° C.

88.2mg of D-(-)-cyclohexadienylaminoacetylchloride is added therein over 30minutes, subsequently the solution is stirred at 0° C for 2.5 hours and then distilled under reduced pressure in order to eliminate the solvent. 2ml of water and 10ml of ethylacetate are added and dissolved in the residue, this is cooled to 5° C and adjusted to pH 6.5~7.0 with saturated sodium bicarbonate. After that, the organic layer is separated and the aqueous layer is extracted with 10ml of ethylacetate. The seperated organic layer and the extract from the water layer are combined together and washed with 5ml of saturated saltwater, dried using magnesium sulfate anhydride and filtered.

The solvent is eliminated, the residue is dissolved in a minimum volume of ethylacetate and passed through a silica gel-column(eluent; toluene/ethylacetate = 6/4). Finally, the solvent is disstilled once again, the residue is crystallized with ethylenechloride and petroleum ether. Consequently, 90mg of pure diphenylmethyl 7-cyclohexadienylaminoacetamido-3-isopropenyl-3-cephem-4-carboxylate are obtained.

NMR($CDCl_3$, $\delta$) : 1.42 (s, $-NH_2$), 1.78 (s, $-CH_3$)

2.65 (s, 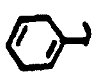), 3.40 (ABq, $C_2$-H)

4.30 (s, $CH-\overset{O}{\overset{\|}{C}}-$), 4.70 (s, $>C=CH_2$)

5.01 (d, 6-H), 5.20 (dd, 7H)

5.70 (s, 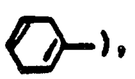), 6.85 (s, $-O-CH<$)

7.30 (s, 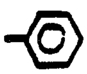 ), 8.10 (d, -NH-)

Example 21 : Preparation of diphenylmethyl 7-p-hydroxyphenylaminoacetamido-3-isopropenyl-3-cephem-4-carboxylate

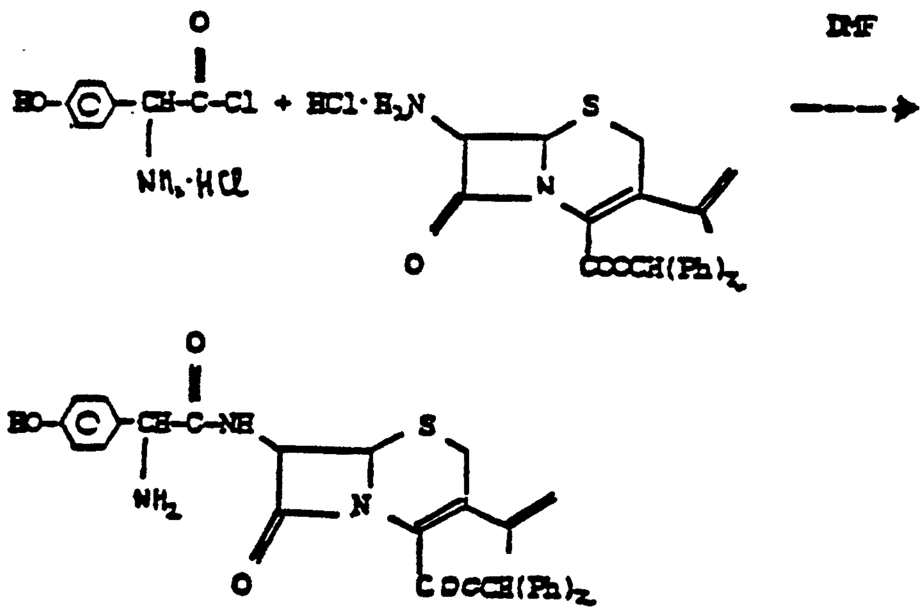


155mg of diphenylmethyl 7-amino-3-isopropenyl-3-cephem-4-carboxylate hydrochloride obtained from Example 12 are dissolved in N,N-dimethylformamide 1.5ml and the solution is cooled to 0°C.

87mg of D-(-)-p-hydroxyphenylaminoacetylchloride hydrochloride are added therein over 30minutes, subsequently the solution is stirred at 0°C for an additional 2.5 hours and distilled under reduced pressure in order to eliminate the solvent. 2ml of water and 15ml of ethylacetate are added and dissolve the residue, the solution is cooled to 5°C and adjusted to pH 6.5~7.0 with saturated sodium bicarbonate. After that, the organic layer is separated and the aqueous layer is extracted with 10ml of ethylacetate. The organic layer and the extract from the water layer are combined together and washed with saturated 5ml of brine dried with magnesium sulfate anhydride and filtered.

The solvent is eliminated, the residue is dissolved in a minimum volume of ethylacetate and passed through a silica gel-column(eluent; toluene/ethylacetate = 1/1). Finally, the solvent is disstilled once again, the residue is crystallized with ethylacetate and petroleum ether. Consequently, 95mg of pure diphenylmethyl 7-hydroxyphenylaminoacetamido-3-isopropenyl-3-cephem-4-carboxylate, which are pure, are obtained.

NMR($CDCl_3$, δ) : 1.42 (s, $-NH_2$), 1.78 (s, $-CH_3$)

3.40 (ABq, $C_2$-H), 4.70 (s, C=CH)

4.48 (s, 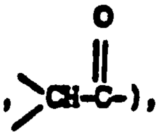),

5.01 (d, 6-H), 5.20 (dd, 7-H),

6.65, 7.16 (d, d, 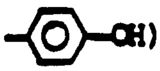),

6.85 (s, 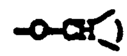),

7.30 (s, 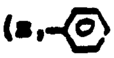), 8.05 (d, -NH-)

Example 22 : Preparation of diphenylmethyl 7-phenylaminoacetamido-3-isopropenyl-3-cephem-4-carboxylate

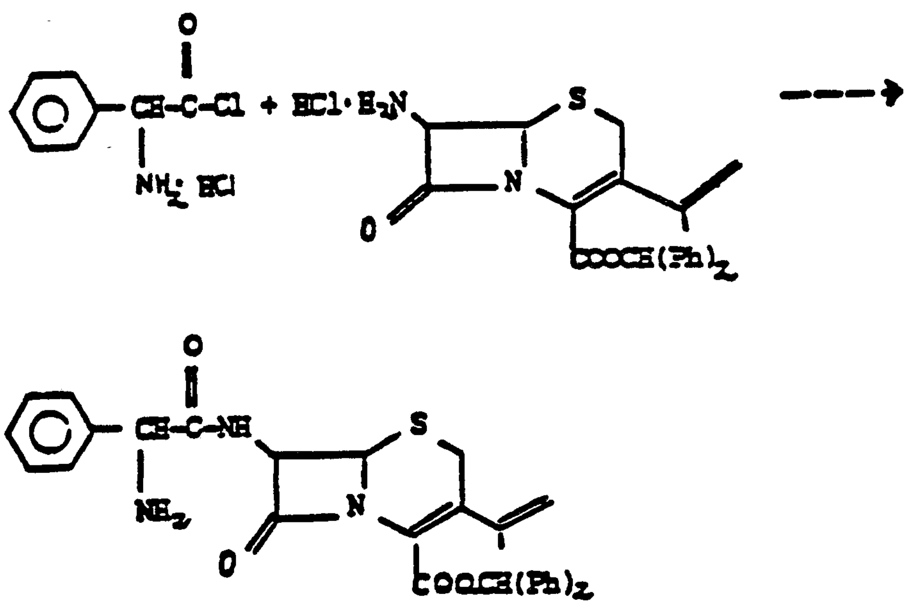

155mg of diphenylmethyl 7-amino-3-isopropenyl-3-cephem-4-carboxylate hydrochloride obtained from Example 12 are dissolved in N,N-dimethylformamide 2.0ml and the resulting solution is cooled to -10° C, and then triethylamine 0.14 ml and ethylalcohol 0.4ml are added therein (solution A).

In the another flask, 90.5mg of D-(-)-phenylaminoacetylchloride hydrochloride are dissolved in 1.2ml of methylenechloride cooled to -50° C and mixed with solution A.

The mixture is respectively stirred at -15° C for 1 hour and at -20° C for 1 hour and at room temperature for 30 minutes.

Methylenechloride is eliminated from the mixture, the residue is added to 20ml of water while stirring. After it has been stirred for 10 minutes, a coarse crystal, obtained by means of filtration, is dissolved in a minimum volume of ethylacetate, and is passed through a silica gel-column (eluent;toluene/ethylacetate

=1/1)

The solvent is eliminated, and the residue is crystallized with ethylacetate and petroleum ether. Consequently, 85mg of pure diphenylmethyl 7-phenylaminoacetamido-3-isopropenyl-3-cephem-4-carboxylate, are obtained.

**NMR($CDCl_3$, δ) : 1.42 (s, $-NH_2$), 1.78 (s, $-CH_3$)**

**3.40 (ABq, $C_2$-H), 4.70 (s, >CH-C(=O)-)**

**4.70 (s, $C=CH_2$), 5.01 (d, 6-H),**

**5.20 (dd, 7-H), 6.85 (s, -O-CH<),**

**7.30 (s, - ), 8.10 (d, -NH-)**

Example 23: Preparation of 7-p-Hydroxyphenylacetamido-3-(2-propenyl)-3-cephem-4-carboxylic acid

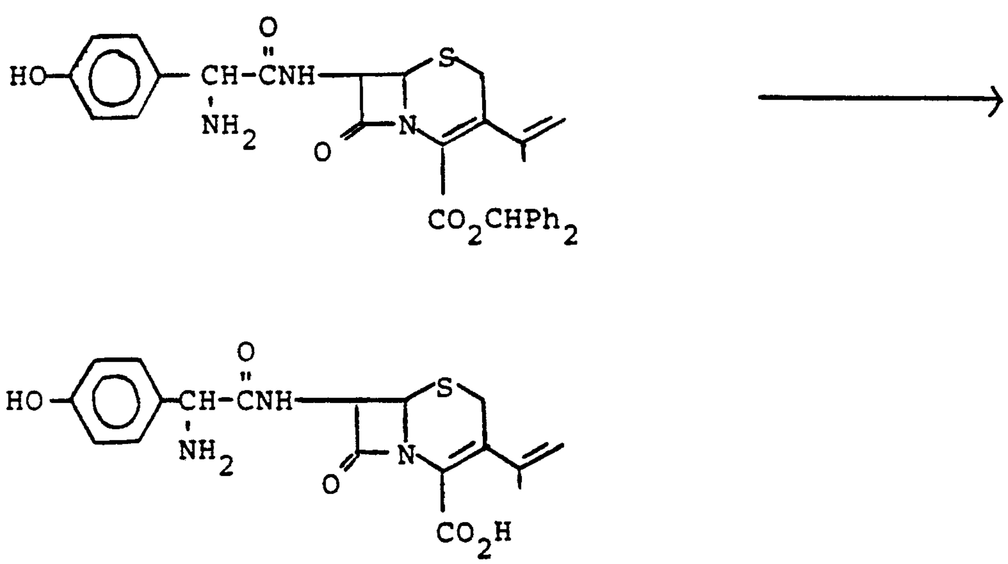

A solution of diphenylmethyl 7-p-hydroxyphenylaminoacetamido-3-(2-propenyl)-3-cephem-4-carboxylate (150 mg, 0.27 mmol), obtained according to Example 21, in formic acid (4 ml) was treated with triethylsilane (0.1 ml) at room temperature for 3 hours. Then the mixture was concentrated and the residue was dissolved in ethyl acetate (20 ml). The solution was extracted with 10 % aqueous bicarbonate. Ethyl acetate (20 ml) was added to the bicarbonate extract and the pH was adjusted to 2 with 1N HCl. The organic layer was separated and diluted with ether. The precipitated solid was filtered to give the title compound (65 mg, 62 %).

1H NMR (DMSO-$d_6$): δ = 1.72 (s, 3H, $CH_3$), 3.45 (d, 2H, C-2),
4.65 (d, 2H, $C=CH_2$), 4.94 (d, 1H, C-6),
5.13 (bs, 1H, CH), 5.66 (s, 1H, OH),
5.78 (dd, 1H, C-7), 6.78 (d, 1H, $NH_2$),
6.67~7.10 (m, 4H).

Example 24: Preparation of 7-Phenylaminoacetamido-3-(2-propenyl)-3-cephem-4-carboxylic acid

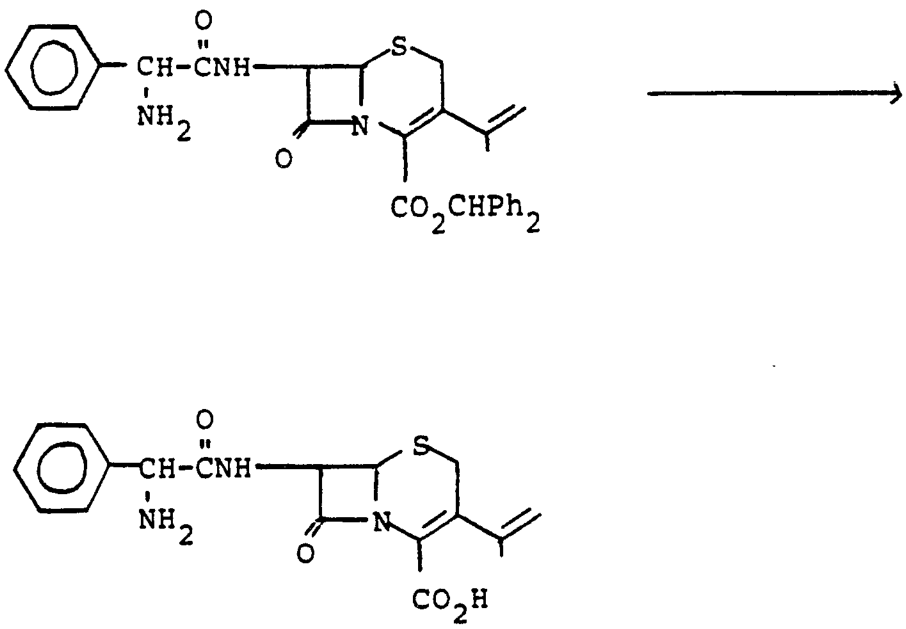

Similarly to Example 23, the deprotection of diphenylmethylester of diphenylmethyl 7-phenylaminoacetamido-3-(2-propenyl)-3-cephem-4-carboxylate, obtained according to Example 22, with triethylsilane in formic acid gave the title compound in 60 % yield.

$^1$H NMR (DMSO-$d_6$): $\delta$ = 1.77 (s, 3H, $CH_3$), 3.44 (d, 2H, C-2), 4.20 (s, 1H, CHC=O), 4.69 (s, 2H, C=$CH_2$), 5.02 (d, 1H, C-6), 5.90 (dd, 1H, C-7), 7.30 (s, 5H, Ph), 8.15 (d, 1H, NH).

Example 25: Preparation of 7-Cyclohexydienylaminoacetamido-3-(2-propenyl)-3-cephem-4-carboxylic acid

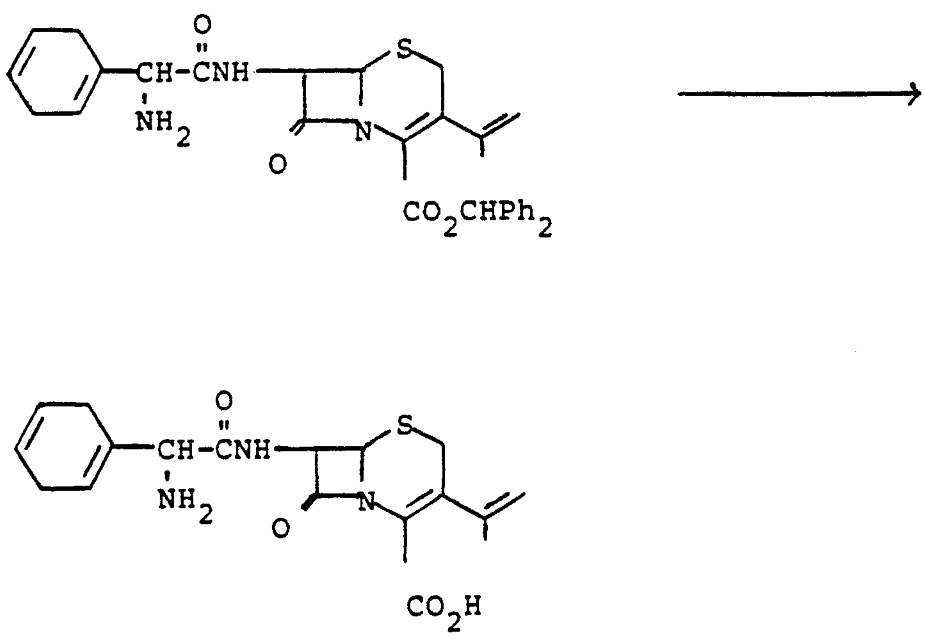

Similarly to Example 23, the deprotection of diphenylmethylester of diphenylmethyl 7-cyclohexadienylaminoacetamido-3-(2-propenyl)-3-cephem-4-carboxylate, obtained according to Example 20, with triethylsilane in formic acid gave the title compound in 55 % yield.

$^{1}H$ NMR (DMSO-$d_6$): $\delta$ 1.78 (s, 3H, $CH_3$), 2.65 (s, 4H), 3.40 (d, 2H, C-2), 4.30 (s, 1H, CHC=O), 4.75 (s, 2H, $C=CH_2$), 5.01 (d, 1H, C-6), 5.70 (s, 3H), 6.00 (dd, 1H, C-7), 5.70 (s, 3H), 7.30 (s, 5H, Ph), 8.10 (s, 2H, $NH_2$).

An alternative, very suitable method for the manufacture of the intermediate (9) [Example 9] starting with compound (5′) [Example 4] is described in Examples 26 and 27.

Example 26: Preparation of Diphenylmethyl 7-phenylacetamido-3-acetyl-2-cephem-4-carboxylate

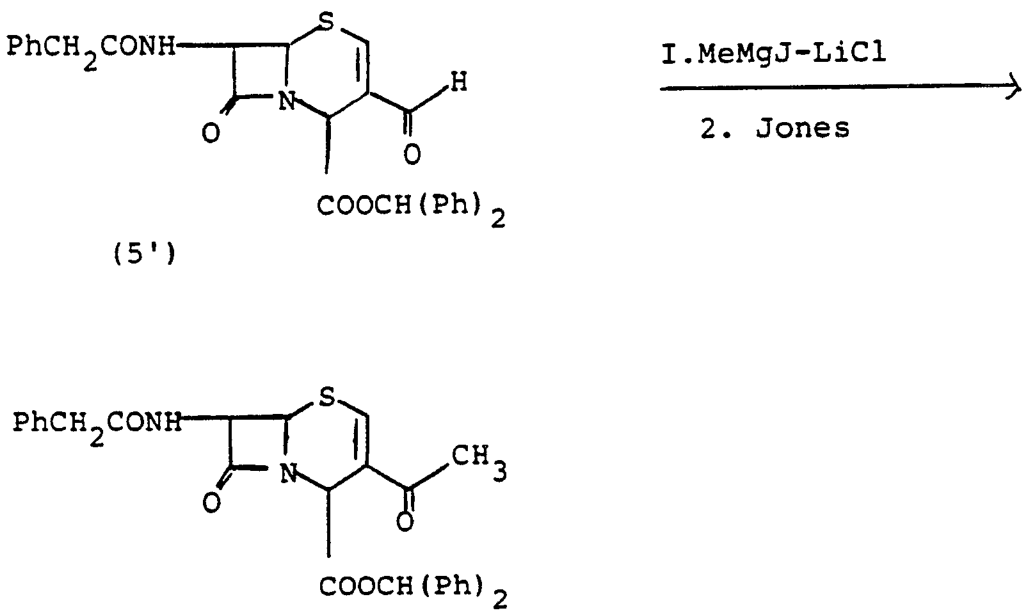


(5')

(8)

A mixture of diphenylmethyl 7-phenylacetamido-3-formyl-2-cephem-4-carboxylate (40 g, 0.08 mole) and lithium chloride (26 g, 8 eq) in dry THF (900 ml) was treated with 5M methylmagnesium iodide (300 ml, 8 eq) for 5 min at -78°C. After 30 min's additional stirring, the mixture was quenched with 300 ml of 1N HCl at -78°C and the pH was adjusted to 3. Thyl acetate extraction followed by concentration gave a crude methyl carbinol as a mixture of two diastereomers. This alcohol, dissolved in acetone (500 ml) was oxidized with Jone's reagent at -10°C. The excess oxidant was quenched with 2-propanol and acetone was removed by rotary evaporation. The residue was extracted with ethyl acetate. The extract was washed with brine, dried ($Na_2SO_4$) and concentrated to give an oil, which upon purification by chromatograph yielded 21.4 g of the title compound in 52 % yield.

$^1H$ NMR ($CDcl_3$): $\delta$ = 2.18 (s, 3H, $COCH_3$), 3.58 (s, 2H, $CH_2Ph$),
4.80 (d, 1H, C-6),5.30 (dd, 1H, C-7),
5.50 (s, 1H, C-4), 6.45 (d, 1H, NH), 6.50
(s, 1H, $CHPh_2$), 7.30 (s, 15H, Ph) and
7.50 (s, 1H, C-2).

Example 27: Preparation of Diphenylmethyl 7-phenylacetamido-3-(2-propenyl)-2-cephem-4-carboxylate

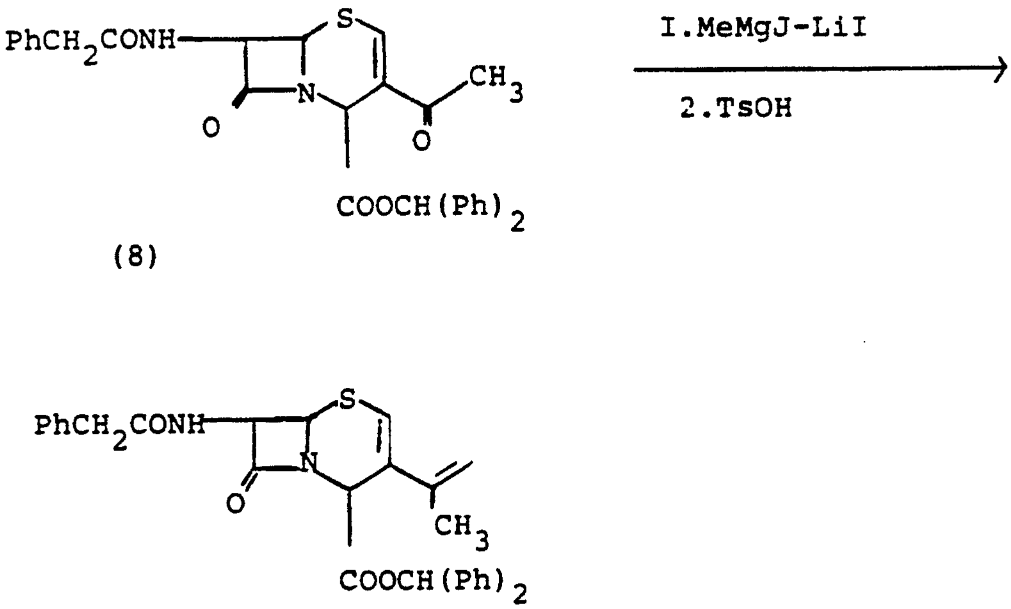


(8)

(9)

A mixture of diphenylmethyl 7-phenylacetamido-3-acetyl-2-cephem-4-carboxylate (20 g) and lithium chloride (16 g) in dry THF (1 l) was treated with 2M methylmagnesiumiodide (190 ml, 10 eq) for 5 min at -78 °C. After stirring for 30 min the mixture was acidified with 1N HCl to pH 3 and extracted with ethyl acetate. The extract was washed with sodium thiosulfate solution, dried ($Na_2SO_4$) and concentrated to give a crude dimethyl carbinol. This carbinol in dichloromethane (200 ml) was treated with catalytic amount of p-toluenesulfonic acid and the progress of the reaction was followed by TLC. After completion of the reaction, the solvent was removed and the residue was subjected to chromatograph to give 12 g of the title compound in 60 % yield.

$^1H$ NMR ($CDCl_3$): $\delta$ = 1.80 (s, 3H, $CH_3$), 3.60 (s, 2H, $CH_2Ph$),
4,80 (d, 2H, $C=CH_2$), 5.10 (d, 1H, C-6),
5.30 (s, 1H, C-4), 5.50 (dd, 1H,C-7), 6.30
(d,1H, NH), 6.40 (s, 1H, C-2), 6.80 (s, 1H, $CHPh_2$)
and 7.30 (s, 15H, Ph).

## Claims

1. A cephem derivative represented by the general formula I:

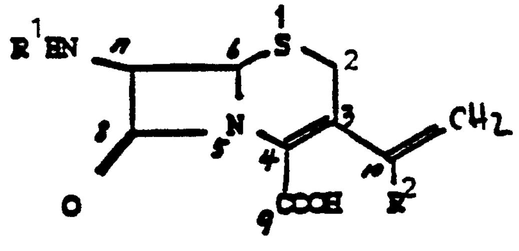

(I)

or a phsiologically acceptable salt thereof,
wherein $R^1$ is a group of the formula A

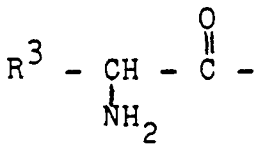

(A)

wherein $R^3$ is a phenyl, monosubstituted phenyl, or a cyclohexadienyl group of the formula

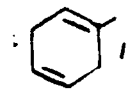

or is a group of the formula B

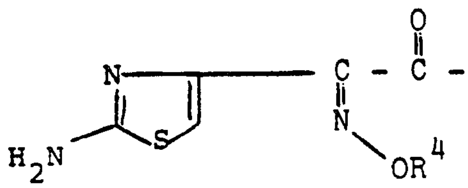

(B)

wherein the $R^4O$-group is in syn-position and $R^4$ denotes $-CH_3$, $-CH_2COOH$ or $-C(CH_3)_2COOH$ and $R^2$ is $C_1$-$C_4$-alkyl or phenyl.

2. Process for the manufacture of compounds of the general formula I and their physiologically acceptable salts, which comprises reacting a compound of the general formula II

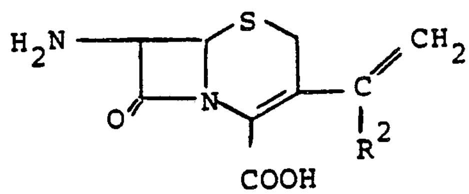

(II)

wherein $R^2$ has the meaning given above and wherein the carboxyl group is in protected form, with a reactive derivate of a compound of the general formulae IIIa or IIIb

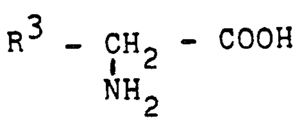

(IIIa)

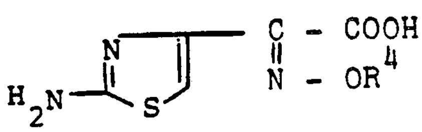

(IIIb)

wherein $R^3$ and $R^4$ have the meaning given above and the $R^4O$-group is in syn-position, wherein the amino group in formula IIIb may also be protected and
wherein a carboxyl group in $R^4$ carries a protecting group and
splitting off any protective group present in the obtained compound of the formula I and - if desired - converting a carboxylic acid of the formula I into a physiologically acceptable salt.

3. Compounds of the general formula IIa

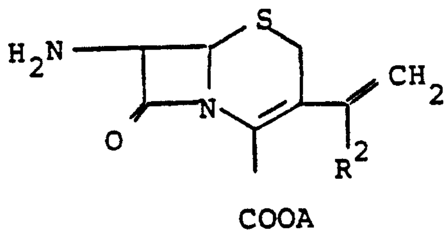

(IIa)

wherein $R^2$ has the meaning given above and A stands for hydrogen or a carboxylic acid protective group.

4. A pharmaceutical preparation effective against bacterial infections, which contains a cephem derivative of the formula I.

5. A process for the preparation of a pharmaceutical preparation effective against bacterial infections, which comprises converting a cephem derivative of the formula I, where appropriate with pharmaceutically customary excipients or diluents, into a pharmaceutically suitable administration form.

6. The use of a cephem derivative of the formula I for controlling bacterial infections.

Claims for the following contracting states: ES, GR

1. A process for the manufacture of cephem derivatives of the general formula I

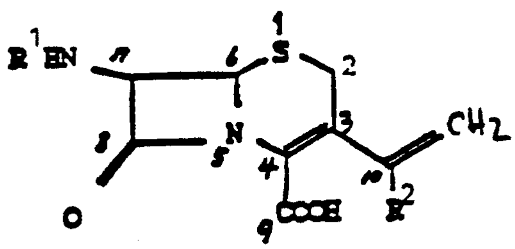

(I)

and their physiologically acceptable salts,
wherein $R^1$ is a group of the formula A

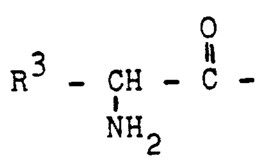

(A)

wherein $R^3$ is a phenyl, monosubstituted phenyl, or a cyclohexadienyl group of the formula

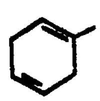 ,

or is a group of the formula B

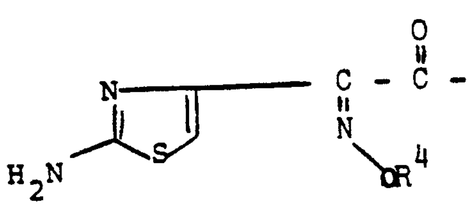

(B)

wherein the $R^4O$-group is in syn-position and $R^4$ denotes $-CH_3$, $-CH_2COOH$ or $-C(CH_3)_2COOH$ and $R^2$ is

$C_1$-$C_4$-alkyl or phenyl,
which comprises reacting a compound of the general formula II

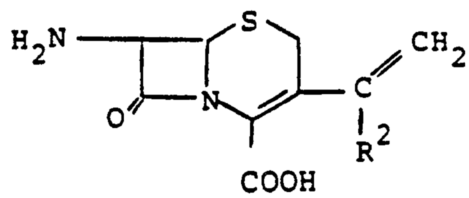

(II)

wherein $R^2$ has the meaning given above and wherein the carboxyl group is in protected form, with a reactive derivate of a compound of the general formulae IIIa or IIIb

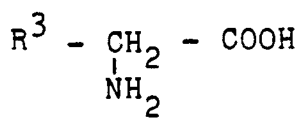

(IIIa)

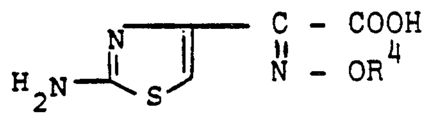

(IIIb)

wherein $R^3$ and $R^4$ have the meaning given above and the $R^4$O-group is in syn-position, wherein the amino group in formula IIIb may also be protected and
wherein a carboxyl group in $R^4$ carries a protecting group and
splitting off any protective group present in the obtained compound of the formula I and - if desired - converting a carboxylic acid of the formula I into a physiologically acceptable salt.

2. A process for the preparation of a pharmaceutical preparation effective against bacterial infections, which comprises converting a cephem derivative of the formula I, where appropriate with pharmaceutically customary excipients or diluents, into a pharmaceutically suitable administration form.